# FASCICULE DE BREVET EUROPEEN

(11) **EP 2 986 255 B1**
(45) Date de publication et mention de la délivrance du brevet: **25.01.2017**
(21) Numéro de dépôt: 14721266.6
(22) Date de dépôt: 17.04.2014
(51) Int. Cl.: A61F 2/24

(54) **IMPLANT, DESTINÉ À ÊTRE PLACÉ DANS UN PASSAGE DE CIRCULATION DE SANG, COMPORTANT UN SYSTÈME D'ÉCARTEMENT DES BRAS PROXIMAUX**
IMPLANTATE ZUM EINSATZ IN EINER BLUTZIRKULATIONSPASSAGE MIT EINEM SYSTEM ZUM TRENNEN DER PROXIMALEN ARME
IMPLANT, INTENDED TO BE PLACED IN A BLOOD CIRCULATION PASSAGE, COMPRISING A SYSTEM FOR SEPARATING THE PROXIMAL ARMS

(30) Priorité: 19.04.2013 FR 1353605
(43) Date de publication de la demande: 24.02.2016
(73) Titulaire: Laboratoires Invalv, 80480 Dury (FR)
(72) Inventeur: RIGHINI, Giovanni, 1196 Gland (CH)
(74) Mandataire: Lavoix
(86) Numéro de dépôt international: PCT/EP2014/057972
(87) Numéro de publication internationale: WO 2014/170463

(56) Documents cités:
- WO-A1-2009/053497
- WO-A1-2013/072496
- US-A1- 2011 313 515

## Description

La présente invention concerne un implant perfectionné destiné à être placé dans un passage de circulation de sang, notamment dans une valve auriculo-ventriculaire cardiaque.

Un tel implant est notamment destiné au remplacement d'une valve cardiaque native, en particulier d'une valve mitrale ou d'une valve tricuspide.

Dans ce cas d'une valve mitrale, l'implant est destiné à être placé dans un passage sanguin d'une valve auriculo-ventriculaire d'un coeur humain ou animal.

Lors de la systole, le passage sanguin entre l'oreillette gauche et le ventricule gauche du coeur est interrompu par la fermeture d'une valve cardiaque native présente dans un appareil mitral. Cette valve assure une circulation univoque du flux sanguin, évitant un reflux à l'issue de la contraction ventriculaire.

L'appareil mitral comprend un anneau mitral, deux feuillets valvulaires reliés à cet anneau, et un appareil sous-valvulaire comprenant des cordages et des piliers. Les feuillets valvulaires comportent un feuillet antérieur, également appelé « grande valve mitrale », et un feuillet postérieur, également appelé « petite valve mitrale ».

La partie de liaison de l'anneau avec la grande valve mitrale est fibreuse, alors que la partie de liaison de l'anneau avec la petite valve mitrale est musculaire. Les petite et grande valves mitrales sont reliées à la partie ventriculaire par des cordages, eux-mêmes reliés aux piliers. En diastole, les deux feuillets s'ouvrent pour libérer le passage entre l'oreillette et le ventricule gauche.

En systole, la contraction ventriculaire engendre une brusque élévation de la pression intra-ventriculaire gauche, provoquant l'éjection du sang à travers la valve aortique. Simultanément, la contraction des piliers et la mise en tension des cordages provoquent la jonction des feuillets entre eux, de façon à isoler de manière étanche les cavités atriale et ventriculaire gauches.

Toutefois, des maladies affectent les valves et les cordages. En particulier, celles-ci peuvent souffrir d'une dégénérescence autorisant ainsi un reflux ou une régurgitation.

De plus, en cas de régurgitation mitrale sévère et chronique, le ventricule gauche sous-jacent se dilate et sa contractivité diminue, ce qui peut conduire à la nécessité d'une intervention chirurgicale cardiaque, même en l'absence de tout symptôme.

Afin de remédier à ces problèmes, il est connu d'implanter un implant entre les feuillets délimitant la valve malade. L'implant comporte par exemple une endoprothèse tubulaire déployable et un obturateur souple réalisé dans un tissu d'origine animal. L'obturateur souple est fixé à demeure dans l'endoprothèse.

De tels implants sont généralement implantables de manière moins invasive qu'un remplacement valvulaire chirurgical, ce qui limite les risques associés à l'implantation de la valve, notamment en termes de mortalité.

On connaît par exemple, d'après WO 2010/121076, un implant mitral disposé dans un passage sanguin auriculo-ventriculaire en remplacement de la valve native.

Un tel implant comporte une pluralité de bras atriaux (également appelés « bras distaux »), et une pluralité de bras ventriculaires (également appelés « bras proximaux ») disposés en regard des bras atriaux pour pincer l'anneau mitral, en prenant appui sur la face atriale des feuillets de la valve native en la plicaturant. Les bras ventriculaires sont formés par des crochets disposés à l'extrémité ventriculaire de l'armature et repliés vers l'extrémité atriale. Les bras atriaux sont formés par des boucles en forme de V s'étendant en regard des bras ventriculaires, au voisinage de ceux-ci, mais s'écartant de l'armature et des bras atriaux.

Les extrémités des bras ventriculaires et des bras atriaux sont disposées à l'écart l'une de l'autre et sont enfoncées respectivement dans une face atriale et dans une face ventriculaire de l'anneau mitral.

Un autre exemple d'implant est notamment décrit dans WO 2011/057087.

US2011/313515 décrit un implant correspondant au préambule de la revendication 1.

On notera que l'installation d'un implant mitral en remplacement de la valve native peut être réalisée en passant par la cavité atriale, ou en passant en variante par la cavité ventriculaire. Cette installation est généralement réalisée au moyen d'un outil de largage adapté. La structure de cet outil de largage peut être différente en fonction du côté (atrial ou ventriculaire) par lequel on passe pour réaliser cette installation.

L'invention a notamment pour but de faciliter l'installation d'un implant mitral, notamment lorsque celui-ci est installé en passant par la cavité ventriculaire.

A cet effet, l'invention a notamment pour objet un implant destiné à être placé dans un passage de circulation de sang, et à être fixé sur un tissu, et comprenant :
- un manchon proximal, de forme générale tubulaire autour d'un axe central, s'étendant longitudinalement entre une extrémité proximale et une extrémité distale, le manchon proximal étant déployable entre une configuration contractée et une configuration déployée,
- une pluralité de bras proximaux, s'étendant chacun entre une première extrémité liée à l'extrémité distale du manchon proximal, et une seconde extrémité libre destinée à s'appuyer sur une première face du tissu, chaque bras proximal s'étendant dans la direction de l'axe central de sorte que son extrémité libre soit disposée au-delà de l'extrémité distale du manchon proximal,
- un manchon distal, de forme générale tubulaire autour de l'axe central, déployable entre une configuration contractée et une configuration déployée, destiné à être assemblé avec le manchon proximal pour former ensemble une armature tubulaire, cette armature définissant un conduit interne de circulation du sang lorsque le manchon proximal et le manchon distal sont assemblés et chacun en configuration déployée,
- une pluralité de bras distaux, portés par le manchon distal, et s'étendant sensiblement perpendiculairement à l'axe central dans la configuration déployée, destinés à s'appuyer sur une deuxième face du tissu, de sorte que le tissu soit alors pincé entre les bras proximaux et les bras distaux,
caractérisé en ce que :
- au moins un bras proximal est déformable élastiquement, entre une position d'écartement et une position d'ancrage, telles que, en l'absence de sollicitation extérieure, la distance radiale entre son extrémité libre et son extrémité liée est supérieure dans la position d'écartement plutôt que dans la position d'ancrage, ce bras proximal étant rappelé élastiquement vers sa position d'ancrage,
- l'implant comporte des moyens de maintien dudit au moins un bras proximal déformable dans sa position d'écartement, comprenant :
   - une première butée, portée par ce bras proximal, et
   - une seconde butée, destinée à coopérer avec la première butée lorsque le manchon proximal est en configuration contractée pour maintenir ce bras proximal dans sa position d'écartement, et à libérer cette première butée lorsque le manchon proximal est en configuration déployée pour laisser le bras proximal se déplacer vers sa position d'ancrage.

Chaque bras proximal s'étend depuis l'extrémité distale du manchon proximal au-delà de cette extrémité distale. Ainsi, lorsque le manchon proximal est approché des feuillets valvulaires depuis la cavité ventriculaire, les bras proximaux se trouvent en avant de ce manchon proximal, si bien qu'ils peuvent accueillir les feuillets valvulaires sans être gênés par ce manchon proximal.

Ainsi, la structure de l'implant défini ci-dessus permet une grande facilité d'insertion des feuillets valvulaires dans un espace de réception délimité par les bras proximaux.

En outre, grâce aux moyens de maintien, l'écartement des bras proximaux est assuré et optimisé lors de l'installation de l'implant.

Il convient de noter que le manchon proximal et le manchon distal forment ensemble un corps d'implant lorsqu'ils sont assemblés. Ainsi, le corps d'implant n'est considéré comme constitué uniquement lorsque ces manchons proximal et distal sont assemblés.

Un implant selon l'invention peut comporter en outre l'une ou plusieurs des caractéristiques suivantes, prises isolément ou suivant toutes combinaisons techniquement envisageables.
- La première butée du bras proximal s'étend latéralement par rapport à ce bras proximal, cette première butée étant de préférence agencée à proximité de l'extrémité liée de ce bras proximal.
- La seconde butée est portée par le manchon proximal.
- Le manchon proximal est formé d'éléments filiformes disposés en grillage, formant des mailles, par exemple en losange, chaque bras proximal muni d'une première butée étant agencé circonférentiellement sur le manchon proximal entre deux mailles consécutives, la seconde butée correspondante étant portée par au moins l'un des éléments filiformes formant ces deux mailles consécutives, ces éléments filiformes étant rapprochés l'un de l'autre en configuration contractée, de sorte que la seconde butée coopère avec la première butée, et éloignés l'un de l'autre en configuration déployée, de façon à laisser un passage radial pour la première butée, libérant ainsi cette première butée.
- Le manchon proximal porte au moins deux bras proximaux munis chacun d'une première butée, tels que la distance entre la première butée et l'extrémité liée de l'un de ces deux bras proximaux est supérieure à la distance entre la première butée et l'extrémité liée de l'autre de ces deux bras proximaux.
- La première butée d'au moins l'un des bras proximaux est formée par une partie en saillie de ce bras proximal, s'étendant latéralement par rapport à ce bras proximal.
- La première butée d'au moins l'un des bras proximaux est formée par un élément rapporté sur ce bras proximal, par exemple soudé, cet élément s'étendant latéralement par rapport à ce bras proximal.

L'invention concerne également un dispositif de traitement d'un passage de circulation de sang, notamment dans une valve auriculo-ventriculaire cardiaque, caractérisé en ce qu'il comporte un implant tel que défini précédemment, et un outil de largage de cet implant, les manchons proximal et distal étant montés dans leurs configurations contractées dans cet outil de largage.

Un dispositif de traitement selon l'invention peut comporter en outre l'une ou plusieurs des caractéristiques suivantes, prises seules ou en combinaison :
- L'outil de largage comporte une gaine de maintien de chaque bras proximal muni d'une première butée dans sa positions d'écartement, ladite gaine de maintien présente une forme générale de révolution autour de l'axe longitudinal, et est destinée à être disposée autour du manchon proximal en configuration contractée, ladite gaine de maintien comporte des bandes longitudinales séparées par des ouvertures longitudinales, chaque bande longitudinale étant destinée à passer à travers une ouverture prévue dans un bras proximal respectif, et chaque bande longitudinale comporte une seconde butée destinée à coopérer avec la première butée du bras proximal à travers lequel passe cette bande longitudinale.
- Ladite bande longitudinale est munie d'un bossage, faisant saillie radialement vers l'extérieur de la gaine de maintien, portant ladite seconde butée.

L'invention concerne également un procédé d'installation d'un implant tel que défini précédemment, sur les feuilles d'une valve native, entre une cavité atriale et une cavité ventriculaire d'un coeur, les feuillets présentant une face atriale et une face ventriculaire, le procédé comportant :
- une étape de positionnement des bras distaux dans la cavité atriale,
- une étape de déploiement des bras distaux dans la cavité atriale,
- une étape d'application des bras distaux contre la face atriale des feuillets,
- une étape de déplacement du manchon proximal vers les feuillets, avec les bras proximaux déployés de manière à définir un espace de réception des feuillets, afin d'insérer les feuillets dans cet espace de réception, et d'application des bras proximaux contre la face ventriculaire des feuillets en appliquant une force axiale vers ces feuillets,
- une étape de déploiement du manchon distal et du manchon proximal, et d'assemblage du manchon distal avec le manchon proximal de manière à former une armature tubulaire de l'implant.

Avantageusement, le procédé utilise un outil de largage dans lequel l'implant est logé en position contractée, l'outil comportant :
- une gaine intérieure pour retenir le manchon distal en configuration contractée, et retenir les bras distaux en position axiale,
- une gaine extérieure pour retenir le manchon proximal en configuration contractée.

Dans ce cas, le procédé comporte :
- une étape de déploiement des bras proximaux, notamment en rétractant la gaine extérieure de manière à libérer les bras proximaux,
- une étape de positionnement des bras distaux dans la cavité atriale, alors que la gaine intérieure est toujours positionnée autour des bras distaux,
- une étape de déploiement des bras distaux dans la cavité atriale, en rétractant la gaine intérieure de manière à libérer ces bras distaux,
- une étape d'application des bras distaux contre la face atriale des feuillets, en déplaçant l'outil vers la cavité ventriculaire de manière à appliquer un effort axial vers cette cavité ventriculaire,
- une étape de déplacement du manchon proximal vers les feuillets, alors que la gaine extérieure recouvre le manchon proximal, et d'application des bras proximaux contre la face ventriculaire des feuillets pour appliquer un effort axial orienté vers la cavité atriale,
- une étape de déploiement des manchons proximal et distal, en rétractant les gaines intérieure et extérieure.

Conformément à un mode de réalisation avantageux:
- l'étape de positionnement des bras distaux dans la cavité atriale fait suite à l'étape de déploiement des bras proximaux,
- l'étape de déploiement des bras distaux dans la cavité atriale fait suite à l'étape de positionnement des bras distaux dans la cavité atriale,
- l'étape d'application des bras distaux contre la face atriale des feuillets fait suite à l'étape de déploiement des bras distaux dans la cavité atriale,
- l'étape de déplacement du manchon proximal vers les feuillets fait suite à l'étape d'application des bras distaux contre la face atriale des feuillets,
- l'étape de déploiement des manchons proximal et distal fait suite à l'étape de déplacement du manchon proximal vers les feuillets.

Conformément à un autre mode de réalisation :
- la gaine extérieure et le manchon proximal en configuration contractée dans la gaine extérieure sont introduits dans la cavité ventriculaire par une première voie, et
- la gaine intérieure et le manchon distal en configuration contractée dans la gaine intérieure sont introduits dans la cavité atriale par une seconde voie différente de la première.

La valve native est choisie entre une valve mitrale ou une valve tricuspide.

L'invention sera mieux comprise à la lecture de la description qui va suivre, donnée uniquement à titre d'exemple, et faite en se référant aux figures annexées, parmi lesquelles :
- la figure 1 est une vue schématique de profil d'un implant selon un exemple de mode de réalisation de l'invention, disposé dans un passage de circulation du sang, dans une valve auriculo-ventriculaire cardiaque ;
- la figure 2 est une vue schématique de profil d'un manchon proximal de l'implant de la figure 1, lorsque ce manchon proximal est disposé dans une gaine d'un outil de largage de l'implant ;
- la figure 3 est une vue schématique de profil du manchon de la figure 2, en configuration contractée, dont les bras proximaux sont en position d'écartement ;
- la figure 4 est une vue schématique de profil du manchon proximal des figures 2 et 3, en configuration déployée ;
- la figure 5 représente un détail du manchon proximal des figures 2 à 4, montrant des moyens de maintien, selon un premier exemple de mode de réalisation, d'un bras proximal dans sa position d'écartement, ces moyens de maintien étant vus de face ;
- la figure 6 est une vue similaire à la figure 5, représentant les moyens de maintien lorsque le manchon proximal est en configuration déployée, et les bras proximaux en position d'ancrage ;
- les figures 7 et 8 sont des vues similaires aux figures 5 et 6, représentant des moyens de maintien, selon un deuxième exemple de mode de réalisation, respectivement lorsque le manchon proximal est en configuration contractée et déployée ;
- les figures 9 et 10 sont des vues similaires aux figures 7 et 8, de moyens de maintien selon un troisième exemple de mode de réalisation, respectivement lorsque le manchon proximal est en configuration contractée et déployée ;
- la figure 11 représente un manchon proximal, représenté déroulé, comprenant des moyens de maintien selon un quatrième exemple de mode de réalisation de l'invention ;
- la figure 12 est une vue schématique en perspective d'un manchon proximal comportant des moyens de maintien selon un cinquième exemple de mode de réalisation de l'invention ;
- la figure 13 est une vue en perspective d'une gaine de maintien des moyens de maintien de la figure 12 ;
- les figures 14 à 20 représentent schématiquement, en coupe axiale, un dispositif de traitement selon l'invention, représenté dans différentes phases de largage de l'implant ;
- les figures 21 à 26 représentent schématiquement, en coupe axiale, un dispositif de traitement selon l'invention, représenté dans différentes phases de largage de l'implant selon un second mode de réalisation du largage ;
- les figures 27 à 32 représentent schématiquement, en coupe axiale, un dispositif de traitement selon l'invention, représenté dans différentes phases de largage de l'implant selon un troisième mode de réalisation du largage.

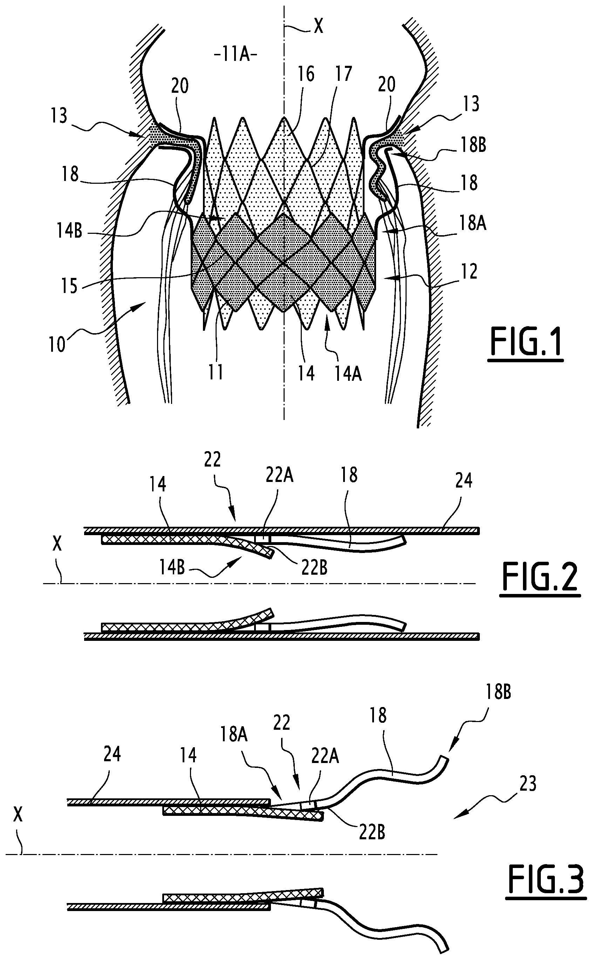

On a représenté, sur la figure 1, un implant 10 destiné à être positionné et déployé dans un passage de circulation de sang, par exemple dans un passage situé dans le coeur d'un patient.

L'implant 10 est avantageusement une valve cardiaque destinée à remplacer une valve native défectueuse, plus particulièrement une valve auriculo-ventriculaire. Ainsi, l'implant 10 est avantageusement destiné à remplacer une valve mitrale native située entre une oreillette gauche 11A et un ventricule gauche 11B du coeur, de façon à permettre une circulation univoque du flux sanguin entre cette oreillette gauche 11 A et ce ventricule gauche 11 B.

En variante, l'implant est une valve atrio-ventriculaire, destinée à remplacer la valve cardiaque en position tricuspide. Il convient de noter que, dans ce cas, l'implant peut être apporté via une voie trans-ventriculaire, ou en variante via une voie trans-jugulaire.

L'implant 10 est notamment destiné à être fixé sur un tissu 13 du coeur, ce tissu 13 étant dans l'exemple représenté formé par des feuillets de valve native.

L'implant 10 comporte une armature tubulaire 12, destinée à définir un conduit interne de circulation de sang. Cette armature 12 est avantageusement munie d'un obturateur (non représenté) à base de tissu, notamment de tissu synthétique ou naturel, tel que bovin, équin et/ou de péricarde porcin. Cet obturateur est destiné à assurer la circulation univoque du sang à travers cette armature 12.

L'armature tubulaire 12 comporte un manchon proximal 14 et un manchon distal 16, rapportés l'un sur l'autre et assemblés pour former l'armature 12.

Il convient de noter que l'armature 12 ne peut être considérée comme formée que lorsque le manchon proximal 14 et le manchon distal 16 sont assemblés.

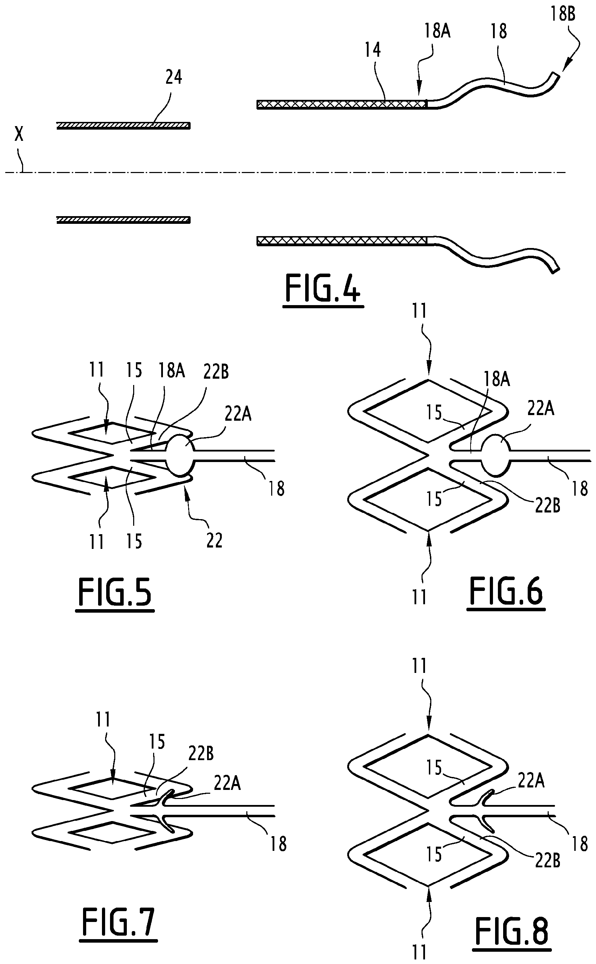

Le manchon proximal 14 présente une forme générale tubulaire autour d'un axe central X. Ce manchon proximal 14 s'étend longitudinalement, dans la direction de l'axe central X, entre une extrémité proximale 14A et une extrémité distale 14B. Ce manchon proximal 14 est déployable entre une configuration contractée (qui sera décrite ultérieurement, notamment en référence aux figures 2 et 3) et une configuration déployée (qui sera décrite ultérieurement, notamment en référence à la figure 4).

L'implant 10 comporte par ailleurs une pluralité de bras proximaux 18, s'étendant chacun entre une première extrémité 18A liée à l'extrémité distale 14B du manchon proximal 14, et une seconde extrémité libre 18B destinée à s'appuyer sur une face proximale d'un feuillet 13 de valve. Le manchon proximal 14 forme donc, avec les bras proximaux 18, un premier ensemble d'un seul tenant.

Chaque bras proximal 18 s'étend dans la direction de l'axe central X de sorte que son extrémité libre 18B soit disposée au-delà de l'extrémité distale 14B du manchon proximal 14.

Les bras proximaux 18 étant destinés à s'appuyer sur le feuillet 13 de valve du côté du ventricule gauche, ces bras proximaux 18 sont également appelés «bras ventriculaires ».

Dans l'exemple de la figure 1, les bras proximaux 18 présente une forme de profil ondulée, de sorte que chaque bras proximal 18 comporte, entre son extrémité liée 18A et son extrémité libre 18B, au moins une région intermédiaire s'étendant le long et à l'écart radialement de l'armature 12 pour définir une loge longitudinale de réception d'un feuillet de valves.

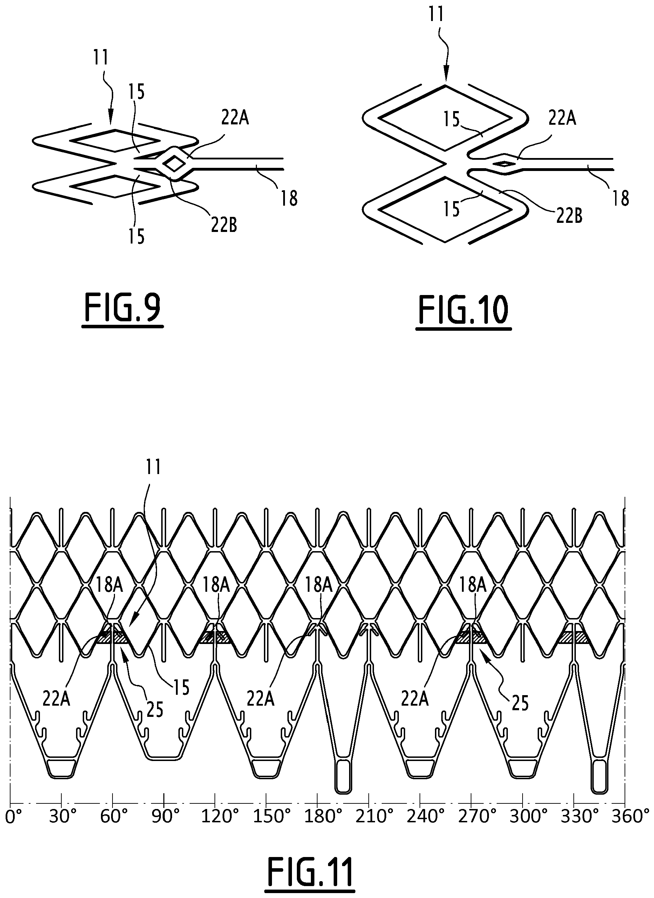

Conformément à une variante préférée, chaque bras proximal 18 comporte deux branches convergeant distalement l'une vers l'autre pour présenter sensiblement une forme de V retourné en configuration déployée. De tels bras proximaux 18 sont notamment représentés sur la figure 11.

Avantageusement, deux bras proximaux 18 adjacents présentent une partie commune depuis laquelle s'étendent une branche respective de chacun de ces bras proximaux. Ainsi, chaque bras proximal 18 comporte deux parties de liaison avec le manchon proximal, chacune de ces parties de liaison étant commune à un bras proximal adjacent respectif, comme cela est représenté sur la figure 11 notamment.

Les parties de liaison d'un bras proximal 18 forment son extrémité liée 18A.

Avantageusement, les formes des bras proximaux 18 sont adaptées à la configuration prédéterminée du passage de circulation de sang destiné à recevoir l'implant 10. Ainsi, par exemple, au moins un bras proximal 18 présente une longueur supérieure à celle d'au moins un autre bras proximal. En variante ou en combinaison avec ladite longueur supérieure, la distance transversale entre les parties de liaison d'au moins un bras proximal est inférieure à celle entre les parties de liaison d'au moins un autre bras proximal.

Le manchon distal 16 présente également une forme générale tubulaire autour de l'axe central X. Ce manchon distal 16 est également déployable entre une configuration contractée et une configuration déployée. Plus particulièrement, le manchon distal 16 est destiné à être assemblé avec le manchon proximal 14 pour former l'armature tubulaire 12 de l'implant 10 lorsque ce manchon proximal 14 et ce manchon distal 16 sont assemblés, chacun en configuration déployée.

L'implant 10 comporte par ailleurs une pluralité de bras distaux 20, chacun étant porté par le manchon distal 16, et s'étendant sensiblement perpendiculairement à l'axe central X lorsque ce manchon distal 16 est dans sa configuration déployée. Ainsi, le manchon distal 16 forme, avec les bras distaux 20, un second ensemble d'un seul tenant, destiné à être rapporté sur le premier ensemble.

Les bras distaux 20 sont destinés à s'appuyer sur une face distale d'un feuillet 13 de valve, c'est-à-dire du côté de l'oreillette gauche, également appelée cavité atriale, lorsque la valve est une valve mitrale. Ainsi, ces bras distaux 20 sont également appelés « bras atriaux ».

Chaque bras distal 20 forme par exemple une boucle faisant saillie transversalement par rapport à l'axe central X.

Lorsque l'implant 10 est installé dans le conduit de circulation de sang, les feuillets 13 de valve sont pincés entre les bras proximaux 18 et les bras distaux 20, assurant ainsi l'ancrage de l'implant 10, comme cela est représenté sur la figure 1.

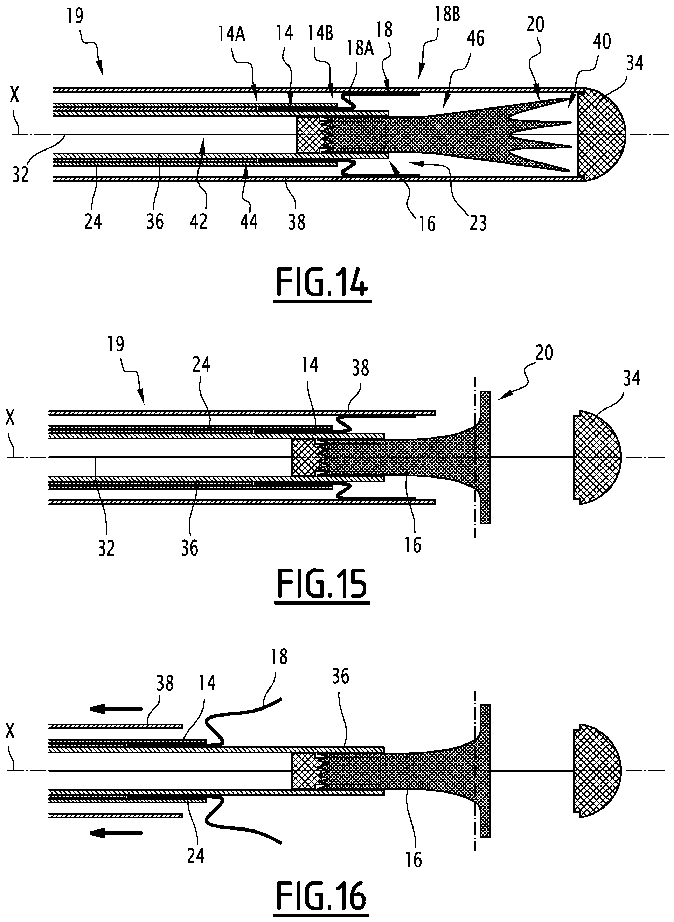

On notera que l'implant 10 est dit «en configuration déployée » lorsque le manchon proximal 14 et le manchon distal 16 sont assemblés en configurations déployées. En revanche, l'implant 10 est dit « en configuration contractée » lorsqu'il est disposé dans un outil de largage 19, dans lequel les manchons proximal 14 et distal 16 sont disposés en configurations contractées, comme cela sera décrit ultérieurement, notamment en référence aux figures 14 à 20.

Avantageusement, chacun des manchons proximal 14 et distal 16, donc également l'implant 10, est auto-expansible, c'est-à-dire que sa configuration déployée constitue sa position de repos. Ainsi, chacun des manchons proximal 14 et distal 16, donc également l'implant 10, dans sa configuration contractée, est sollicité élastiquement vers sa configuration déployée.

Par exemple, le manchon proximal 14, le manchon distal 16, les bras proximaux 18 et les bras distaux 20 sont formés d'un acier inoxydable ayant des propriétés élastiques. En variante, ces éléments sont formés à base de métal à mémoire de forme tel que du nitinol (nickel/titane) ou d'une fibre souple polymère.

Le manchon proximal 14 est par exemple formé par un treillis d'éléments filiformes 15 entrelacés, délimitant des mailles M, par exemple des mailles polygonales, de préférence des mailles en forme de losange.

De même, le manchon distal 16 est par exemple formé par un treillis d'éléments filiformes 17 entrelacés, délimitant des mailles, par exemple des mailles polygonales, de préférence des mailles en forme de losange.

Avantageusement, le manchon proximal 14 présente, lorsqu'il est séparé du manchon distal 16, et en l'absence de sollicitation extérieure, un diamètre inférieur à celui du manchon distal 16 en l'absence de sollicitation extérieure. Ainsi, lorsque le manchon distal 16 est déployé à l'intérieur du manchon proximal 14, il exerce un effort radial sur une surface interne de ce manchon proximal 14, cet effort radial étant suffisant pour assurer la liaison entre le manchon proximal 14 et le manchon distal 16.

Par ailleurs, le manchon distal 16 présente une longueur, dans la direction de l'axe X, supérieure à la longueur du manchon proximal 14. Ainsi, le manchon proximal 14 peut être disposé selon différentes positions sur le manchon distal 16, notamment en fonction de la configuration du passage de circulation de sang destiné à recevoir l'implant 10. A cet effet, les manchons proximal 14 et distal 16 sont déplaçables axialement l'un par rapport à l'autre avant assemblage.

Conformément à l'invention, au moins l'un des bras proximaux 18, de préférence chaque bras proximal 18, est déformable élastiquement, entre une position d'écartement, notamment représentée sur la figure 3, et une position d'ancrage, notamment représentée sur les figures 1 et 4, telles que, en l'absence de sollicitation extérieure, la distance radiale entre son extrémité libre 18B et son extrémité liée 18A est supérieure dans la position d'écartement plutôt que dans la position d'ancrage. On rappellera que cette distance radiale est la différence entre la distance entre l'extrémité libre 18B et l'axe central X, perpendiculairement à cet axe central X, et la distance entre l'extrémité liée 18A et l'axe central X, perpendiculairement à cet axe central X.

Chacun de ces bras proximaux 18 est rappelé élastiquement vers sa position d'ancrage. En revanche, l'implant 10 comporte des moyens 22 de maintien d'au moins l'un de ces bras proximaux 18 déformables dans sa position d'écartement.

Les moyens de maintien 22 comprennent au moins une première butée 22A portée par ce bras proximal 18, et au moins une seconde butée 22B, destinée à coopérer avec la première butée 22A lorsque le manchon proximal 14 est en configuration contractée pour maintenir le bras proximal dans sa position d'écartement, et à libérer cette première butée 22A lorsque le manchon proximal 14 est en configuration déployée.

Un premier exemple de mode de réalisation des moyens de maintien 22 est représenté sur les figures 5 et 6.

Conformément à ce premier mode de réalisation, la première butée 22A de chaque bras proximal 18 est portée par une portion de ce bras proximal 18, s'étendant latéralement par rapport à ce bras proximal 18. Par « extension latérale », on entend que cette portion s'étend notamment dans une direction sensiblement perpendiculaire à la direction générale longitudinale du bras proximal 18, et sensiblement parallèle à l'axe X.

En particulier, conformément à ce premier mode de réalisation, la première butée 22A est formée par un élargissement du bras proximal 18 dans cette direction latérale. Cet élargissement présente par exemple une forme générale ovale.

La première butée 22A est de préférence agencée à proximité de l'extrémité liée 18A de ce bras proximal 18

Par ailleurs, la seconde butée 22B est portée par le manchon proximal 14. Plus particulièrement, chaque bras proximal 18 étant agencé circonférentiellement sur le manchon proximal 14 entre deux mailles M, circonférentiellement consécutives à l'extrémité distale 14B de ce manchon proximal 14, la seconde butée 22B est portée par au moins l'un des éléments filiformes 15 formant ces deux mailles consécutives M.

Ces éléments filiformes 15 sont rapprochés l'un de l'autre en configuration contractée, comme cela est représenté sur la figure 5, de sorte que la seconde butée 22B coopère avec la première butée 22A.

En revanche, ces éléments filiformes 15 sont éloignés l'un de l'autre en configuration déployée, comme cela est représenté sur la figure 6, de façon à laisser un passage radial pour la première butée 22A, libérant ainsi cette première butée 22A.

Ainsi, en configuration contractée du manchon proximal 14, le bras proximal 18 est contraint dans sa position d'écartement. En revanche, dans la configuration déployée, le bras proximal 18 est rappelé élastiquement vers sa position d'ancrage.

A titre illustratif, le manchon proximal 14 est représenté dans différents stades de son déploiement sur les figures 2 à 4.

Sur la figure 2, le manchon proximal 14 est contraint dans sa configuration contractée par une gaine 24 de l'outil de largage 19 de l'implant 10. Cette gaine 24 applique donc une sollicitation extérieure sur les bras proximaux 18 et sur le manchon proximal 14.

Dans cette gaine 24, le manchon proximal 14 étant dans sa position contractée, la première butée 22A de chaque bras proximal 18 est en contact avec la seconde butée 22B correspondante, cette première butée 22A étant située radialement à l'extérieur du manchon proximal 14 par rapport à la seconde butée 22B. Ainsi, chaque bras proximal 18 est contraint vers sa position écartée par les moyens de maintien 22. Toutefois, du fait de la sollicitation extérieure appliquée par la gaine 24, les bras proximaux 18 ne peuvent pas s'écarter vers l'extérieur, si bien que la seconde butée 22B est contrainte vers l'intérieur du manchon distal 14 en réaction.

Sur la figure 3, la gaine 24 a été coulissée longitudinalement dans la direction de l'axe central X, de manière à libérer les bras proximaux 18 du manchon proximal 14. Ceux-ci, en l'absence de la sollicitation extérieure appliquée par la gaine 24, se déploient alors dans leur position d'écartement, sous l'effet de la contrainte exercée par les moyens de maintien 22.

C'est dans cette configuration que le manchon proximal 14 est installé dans le conduit de circulation de sang. En effet, l'écartement des bras proximaux 14 permet notamment de faciliter l'insertion des feuillets 13 de valve dans un espace 23 de réception de ces feuillets de valve, délimités entre les bras proximaux 18. On notera que le manchon proximal 14 s'étend intégralement (ou en variante, en majorité) en dehors de cet espace de réception 23, si bien qu'il ne gêne pas l'insertion des feuillets 13 de valve dans cet espace de réception 23.

Une fois le manchon proximal 14 en place dans le conduit de circulation de sang, la gaine 24 est encore coulissée longitudinalement de manière à dégager le manchon proximal 14. Celui-ci, en l'absence de cette sollicitation extérieure, se déploie en configuration déployée. La figure 4 représente ce manchon proximal libéré, en configuration déployée.

Dans cette configuration déployée, les éléments filiformes 15 des deux mailles M constitutives encadrant chaque bras proximal 18 sont éloignés l'un de l'autre, comme représenté sur la figure 6, laissant un passage radial pour la première butée 22A, libérant ainsi cette première butée 22A. Chaque bras proximal 18 n'est donc plus sollicité par les moyens de maintien 22, et il est donc rappelé élastiquement vers sa position d'ancrage, dans laquelle l'extrémité libre 18B de ce bras proximal 18 vient prendre appui contre l'un des feuillets 13 de valve.

On notera que la première butée 22A peut prendre toute forme envisageable adaptée.

Par exemple, conformément à un deuxième mode de réalisation représenté sur les figures 7 et 8, la première butée 22A est portée par des pattes s'étendant latéralement en saillie du bras proximal 18. Chacune de ces pattes est propre à prendre appui contre une seconde butée 22B formée par un élément filiforme 15 d'une maille M correspondante encadrant ce bras proximal 18.

Conformément à un troisième mode de réalisation représenté sur les figures 9 et 10, la première butée 22A est portée par une forme réalisée par découpage et déformation du bras proximal 18, cette forme s'étendant alors latéralement en saillie de ce bras proximal 18.

Dans ce cas, chaque bras proximal 18 est avantageusement compressé longitudinalement dans sa position d'écartement, comme représenté sur la figure 9, de sorte que la forme découpée s'étende latéralement. En revanche, chaque bras proximal 18 est avantageusement étiré longitudinalement dans sa position d'ancrage, comme représenté sur la figure 10, de sorte que la forme découpée se contracte latéralement. On prévoit alors dans ce cas des moyens adaptés pour comprimer et étirer longitudinalement chaque bras proximal.

Conformément à un quatrième mode de réalisation représenté sur la figure 11, la première butée 22A d'au moins l'un des bras proximaux 18 est formée par un élément 25 rapporté sur ce bras proximal 18, par exemple par soudage, cet élément s'étendant latéralement par rapport à ce bras proximal 18.

Cet élément 25 est par exemple formé par une plaquette métallique soudée sur le bras proximal 18.

Avantageusement, comme représenté sur cette figure 11, la première butée 22A d'un bras proximal 18 peut être disposée plus ou moins éloignée de l'extrémité liée de ce bras proximal 18. Plus particulièrement, le manchon proximal 14 porte au moins deux bras proximaux 18 munis chacun d'une première butée 22A, tels que la distance entre la première butée 22A et l'extrémité liée 18A de l'un de ces deux bras proximaux 18 est supérieure à la distance entre la première butée 22A et l'extrémité liée 18A de l'autre de ces deux bras proximaux 18.

La distance entre la première butée 22A et l'extrémité liée 18A d'un bras proximal 18 est notamment choisie de manière à assurer un maintien optimal de ce bras proximal 18 dans sa position écartée.

Comme il est également possible de le constater sur la figure 11, dans le cas où deux bras proximaux 18 adjacents partagent une partie de liaison commune, chaque première butée 22A est également avantageusement commune à deux bras proximaux adjacents 18, et à cet effet agencée sur ladite partie de liaison commune.

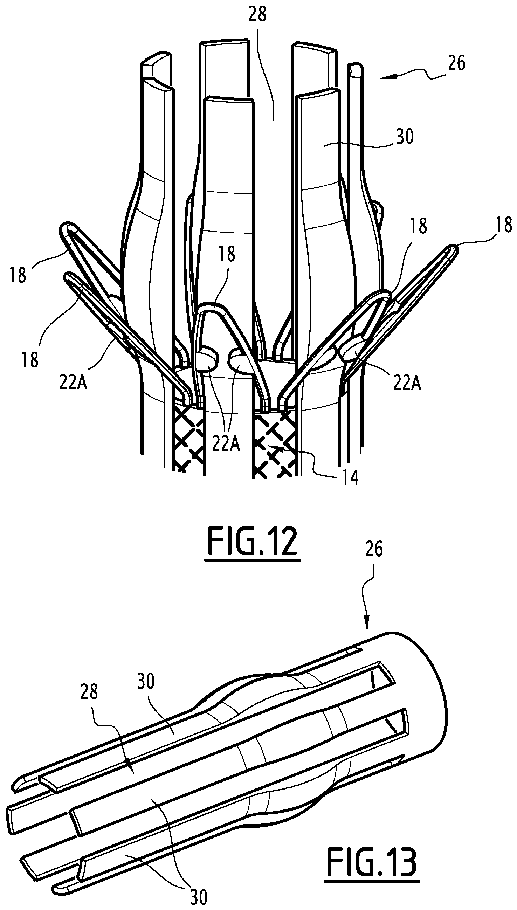

Conformément à un cinquième mode de réalisation, représentée sur les figures 12 et 13, chaque seconde butée 22B est portée par une gaine de maintien 26, représentée sur la figure 13.

Cette gaine de maintien 26 présente une forme générale de révolution autour de l'axe longitudinal X, et elle est destinée à être disposée autour du manchon proximal 14 en configuration contractée, comme représenté sur la figure 12.

Cette gaine de maintien 26 comporte des bandes longitudinales 30 séparées par des ouvertures longitudinales 28. Chaque bande longitudinale 30 est destinée à passer à travers une ouverture prévue dans un bras proximal 18 respectif. Une telle ouverture est par exemple définie entre deux branches du bras proximal 18, lorsque ce bras proximal présente une forme en V telle que représentée sur la figure 12.

Dans ce cas, chaque bras proximal comporte au moins une première butée 22A, s'étendant latéralement vers ladite ouverture, de façon à coopérer avec au moins une seconde butée 22B respective portée par la bande longitudinale 30 passant dans cette ouverture. Chaque première butée 22A est alors portée par un bord de ladite ouverture, par exemple par une branche du bras proximal 18, et s'étend en saillie vers l'intérieur de ladite ouverture.

Il convient de noter qu'une telle gaine de maintien 26 est également compatible avec des bras proximaux présentant des formes similaires à celles des bras proximaux représentés sur la figure 11.

Chaque bande longitudinale 30 est avantageusement munie d'un bossage, faisant saillie radialement vers l'extérieur de la gaine 26, ce bossage portant la seconde butée 22B. Ainsi, en coulissant longitudinalement la gaine, il est possible de faire varier l'épaisseur radiale de ce bossage, donc la distance radiale entre la seconde butée 22B en contact avec la première butée 22A et l'axe central X. Ainsi, il est possible de faire varier l'écartement des bras proximaux 18, afin de s'adapter de manière optimale à la configuration du passage de circulation de sang, notamment en ne déployant les bras proximaux 18 dans leur position d'écartement que lorsque cela est nécessaire.

On notera que la gaine de maintien 26 est par exemple formée par la gaine 24 de l'outil de largage, décrite précédemment en référence aux figures 2 à 4, ou formée par une gaine supplémentaire.

On a représenté, sur la figure 14, un dispositif de traitement, comprenant l'outil de largage 19, recevant un implant 10 tel que défini précédemment. Sur cette figure, et les suivantes, les éléments analogues à ceux des figures précédentes sont désignés par des références identiques.

Comme indiqué précédemment, l'implant 10 comporte un premier ensemble d'un seul tenant comportant le manchon proximal 14 de l'armature et les bras proximaux 18, et un deuxième ensemble d'un seul tenant comportant le manchon distal 16 de l'armature et les bras distaux 20.

Le manchon proximal 14 s'étend, le long de l'axe central X, entre son extrémité proximale 14A et son extrémité distale 14B. L'extrémité liée 18A de chaque bras proximal 18 est liée à l'extrémité distale 14B du manchon proximal 14, et l'extrémité libre 18B de chaque bras proximal 18 s'étend dans la direction de l'axe central X au-delà de cette extrémité distale 14B du manchon proximal 14.

L'outil de largage 19 comporte une tige interne, munie d'une tête 34 de maintien d'une extrémité de l'implant 10, et un tuteur 32 monté coulissant coaxialement sur la tige.

L'outil de largage 19 comporte par ailleurs une gaine intérieure 36, montée coulissante par rapport au tuteur 32, une gaine intermédiaire 24 montée coulissante autour de la gaine intérieure 36, et une gaine extérieure 38 montée coulissante autour de la gaine intermédiaire 24.

On notera que la gaine intermédiaire 24 est celle entourant le manchon proximal 14, comme décrit précédemment en référence aux figures 2 à 4. De même, dans le cas où les moyens de maintien 22 comportent une gaine de maintien 26, comme décrit précédemment en référence aux figures 13 et 14, la gaine intermédiaire est formée par cette gaine de maintien.

Il est également à noter qu'en variante, l'outil de largage 19 pourrait ne comporter, au lieu d'une gaine intermédiaire et une gaine externe, qu'une gaine externe remplissant les fonctions de ladite gaine intermédiaire et de ladite gaine externe.

Le tuteur 32 et les gaines 36, 24, 38 sont déplaçables à coulissement, indépendamment l'une de l'autre, et par rapport à la tige.

Des organes de verrouillage (non représentés) sont généralement prévus entre la tige et le tuteur 32, entre le tuteur 32 et les gaines 36, 24, 38 pour éviter le coulissement spontané du tuteur 32, des gaines externes 38, intermédiaire 24 et internes 36. Ceci permet de procéder par étapes successives aux retraits de la gaine externe 38, de la gaine intermédiaire 24, de la gaine interne 36.

La tête 34 délimite un logement 40 de réception de l'implant 10, dans lequel cet implant 10 est maintenu en configuration contractée.

Plus particulièrement, le tuteur 32 et la gaine interne 36 délimitent entre eux un espace annulaire interne 42, destiné à recevoir le manchon distal 16 et les bras distaux 20. Ainsi, le manchon distal 16 est maintenu en configuration contractée par cette gaine interne 36.

La gaine interne 36 et la gaine intermédiaire 24 délimitent entre elles ensemble un espace annulaire intermédiaire 44, destiné à recevoir le manchon proximal 14. Ainsi, le manchon proximal 14 est maintenu en configuration contractée par cette gaine intermédiaire 24, comme décrit précédemment.

La gaine externe 38 et la gaine intermédiaire 24 délimitent entre elles un espace annulaire extérieur 46, destiné à recevoir les bras proximaux 18. Ainsi, chaque bras proximal 18 est plaqué contre la gaine externe 38 lorsqu'il n'est pas recouvert par la gaine intermédiaire 24, et que le manchon proximal 14 est en configuration contractée, comme représenté sur la figure 3.

Lorsque l'implant 10 doit être positionné, notamment en remplacement d'une valve native, il est introduit entre les feuillets 13 de la valve native autour du siège de la valve. Cette introduction peut être effectuée, à l'aide du dispositif de la figure 14, en passant à travers le ventricule gauche, comme cela sera décrit ci-dessous en référence aux figures 15 à 20.

Comme cela est représenté sur la figure 15, la tête 34 et la partie aval de l'outil 19 sont introduites dans la cavité atriale, au-delà de l'anneau mitral, de sorte que les bras distaux 20 soient disposés dans la cavité atriale au-delà de l'anneau mitral.

La gaine externe 38 est rétractée axialement à l'écart de la tête 34 par rapport à la gaine intermédiaire 24, la gaine interne 36 et par rapport au tuteur 32, pour découvrir les bras distaux 20, qui se déploient alors. L'outil 19 est alors déplacé vers le ventricule gauche pour plaquer les bras distaux 20 contre la face atriale des feuillets 13.

Comme cela est représenté sur la figure 16, la gaine externe 38 est encore rétractée axialement pour découvrir les bras proximaux 18. Ceux-ci, qui étaient maintenus plaqués contre cette gaine externe 38, se déploient alors, sous l'effet de la contrainte imposée par les moyens de maintien 22. En effet, le manchon proximal 14 est maintenu dans sa configuration contractée, et les bras proximaux 18 ne sont soumis à aucune sollicitation extérieure, si bien qu'ils sont alors contraints dans leur position d'écartement par les moyens de maintien 22, comme décrit précédemment.

L'écartement des bras proximaux 18 est alors assuré de manière optimale pour que les feuillets 13 se trouvent en regard d'un espace de réception 23 défini entre ces bras proximaux 18.

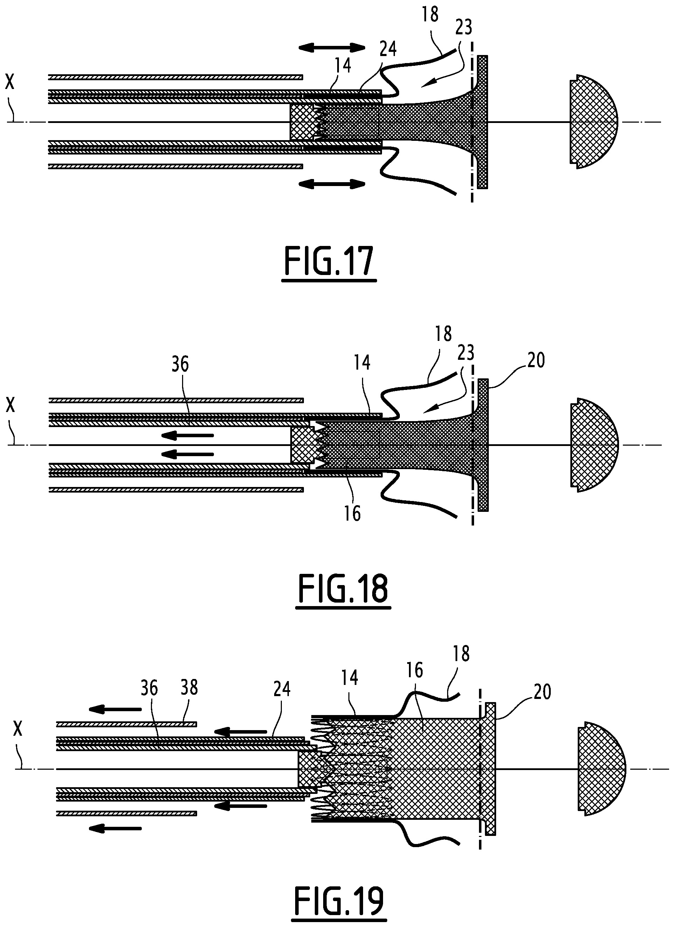

Comme cela est représenté sur la figure 17, il suffit alors de déplacer le manchon proximal 14 et la gaine intermédiaire 24 qui l'entoure, en direction des feuillets 13. Ces feuillets 13 sont ainsi insérés dans l'espace de réception 23 défini entre les bras proximaux 18. Du fait que les bras proximaux 18 s'étendent au-delà de l'extrémité distale 14B du manchon proximal 14, ce manchon proximal 14 ne gêne pas l'insertion des feuillets 13 dans cet espace de réception 23.

Une fois le manchon proximal 14 en place, la gaine interne 36 est rétractée de manière à libérer le manchon distal 16, comme cela est représenté sur la figure 18. Ce manchon distal 16 étant disposé à l'intérieur du manchon proximal 14, il est maintenu en configuration contractée par ce manchon proximal 14, lui-même maintenu en configuration contractée par la gaine intermédiaire 24.

En se déployant à l'intérieur du manchon proximal 14, le manchon distal 16 se lie à ce manchon proximal 14.

On notera que, dans cette position, les feuillets 13 de la valve native sont intercalés entre le manchon distal 16 et les bras proximaux 18.

Une fois les manchons proximal 14 et distal 16 ainsi liés, on rétracte la gaine intermédiaire 24, de manière à libérer le manchon proximal 14, comme cela est représenté sur la figure 19.

Le manchon proximal 14 se déploie radialement alors jusqu'à sa configuration déployée, ainsi que le manchon distal 16 à l'intérieur de ce manchon proximal 14 déployé.

Dans cette configuration déployée du manchon proximal 14, les moyens de maintien 22 relâchent les bras proximaux 18, qui sont alors rappelés élastiquement vers leur position d'ancrage, comme décrit précédemment.

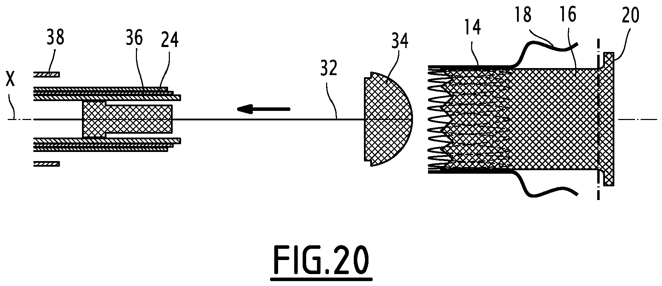

Ceci étant fait, la fixation axiale entre le tuteur 32 et l'extrémité proximale de l'implant 10 est libérée. Le tuteur 32, la tête 34 et la tige sont alors retirés hors du patient à travers le conduit interne, comme cela est représenté sur la figure 20.

On notera que, dans cette configuration déployée, le manchon distal 16 s'étend longitudinalement, en partie à l'intérieur du manchon proximal 14, coaxialement à ce manchon proximal 14, en partie à l'intérieur de l'espace de réception 23, de sorte que le tissu est reçu entre les bras proximaux 18 et le manchon distal 16, et en partie au-delà de l'espace de réception 23. Cette configuration déployée correspond à celle de la figure 1.

Par ailleurs, le manchon proximal 14 est agencé axialement à l'écart des feuillets 13, à l'intérieur de la cavité ventriculaire.

On notera que l'invention n'est pas limitée aux modes de réalisation précédemment décrits.

En particulier, on pourrait prévoir d'autres formes adaptées pour les première et seconde butées des moyens de maintien 22.

Par ailleurs, l'invention peut être appliquée à différentes formes de bras proximaux 18

Par exemple, l'invention pourrait être appliquée à un implant dont chaque bras proximal 18 comprend :
- une partie proximale, partant axialement depuis l'extrémité liée en direction de l'extrémité proximale du manchon proximal, portant la première butée
- une partie de bifurcation, reliant le tronçon proximal à une partie distale du bras proximal
- la partie distale, partant axialement depuis la partie de bifurcation en direction de l'extrémité distale du manchon proximal.

Ladite partie distale peut également présenter une forme bombée.

Dans ce cas, la partie proximale et la partie de bifurcation définissent ensemble une loge plus profonde pour les feuillets 13. Bien évidemment, la longueur de la partie proximale peut être choisie plus ou moins importante, en fonction de la profondeur souhaitée pour la loge.

Conformément à une autre variante, au moins un bras proximal 18 présente une région distale faisant saillie radialement à l'écart de l'axe central X par rapport à une région intermédiaire de ce bras proximal.

Plus particulièrement, au moins un premier bras proximal 18 présente une première région distale appliquée en regard d'un premier bras distal 20, et un deuxième bras proximal 18 présente une deuxième région distale appliquée en regard d'un deuxième bras distal 20, l'étendue radiale de la première région distale étant supérieure à l'étendue radiale de la deuxième région distale.

En termes généraux, l'étendue radiale de la région distale de chaque bras proximal 18 peut être choisie plus ou moins grande, notamment en fonction de la forme prédéterminée du passage de circulation de sang destiné à recevoir l'implant.

Conformément à une autre variante, chaque bras proximal 18 comporte une partie intermédiaire déformable élastiquement selon une direction longitudinale du bras proximal 18. A cet effet, la partie intermédiaire présente généralement la forme d'un ressort.

Grâce à sa partie intermédiaire longitudinalement déformable élastiquement, la longueur de chaque bras proximal 18 peut varier, et s'adapter en fonction du positionnement du manchon proximal 14 par rapport au manchon distal 16, notamment afin d'assurer un pincement optimal des feuillets 13 entre les bras proximaux 18 et les bras distaux 20. En d'autres termes, en l'absence de feuillets 13, il est possible d'assurer un contact entre l'extrémité libre 18B d'au moins un bras proximal 18 et un bras distal 20, dans la configuration déployée et en l'absence de sollicitation extérieure.

Conformément à une autre variante, chaque bras proximal 16 comporte une partie intermédiaire pliée de manière à former un retour axial délimitant une cavité. Cette cavité est destinée à former une loge pour les feuillets 13.

Toute autre forme de bras proximaux compatibles avec des moyens de maintien 22 est envisageable.

On notera par ailleurs que l'implant 10 peut être installé selon un autre procédé d'introduction que celui décrit précédemment. En particulier, les différentes étapes d'installation des manchons proximal 14 et distal 16 peuvent être réalisées dans un autre ordre que celui décrit précédemment.

Par exemple, les bras proximaux 18 peuvent être préalablement déployés, la gaine externe 38 étant rétractée axialement pour découvrir ces bras proximaux 18. Dans ce cas, le manchon proximal 14 est maintenu dans sa configuration contractée, et les bras proximaux 18, qui ne sont soumis à aucune sollicitation extérieure, sont alors contraints dans leur position d'écartement par les moyens de maintien 22, comme décrit précédemment.

L'écartement des bras proximaux 18 est alors assuré de manière optimale pour que les feuillets 13 se trouvent en regard d'un espace de réception 23 défini entre ces bras proximaux 18. Il est à noter qu'en commençant ainsi par installer les bras proximaux 18, leur manipulation n'est pas gênée par le manchon distal 16 et les éléments permettant son installation.

Le manchon proximal 14, ainsi que la gaine intermédiaire 24 qui l'entoure, sont alors déplacés en direction des feuillets 13. Ces feuillets 13 sont ainsi insérés dans l'espace de réception 23 défini entre les bras proximaux 18.

Une fois les bras proximaux 18 convenablement agencés, la tête 34 et la partie aval de l'outil 19 sont alors introduites dans la cavité atriale, au-delà de l'anneau mitral, de sorte que les bras distaux 20 soient disposés dans la cavité atriale au-delà de l'anneau mitral. Dans ce cas, les bras distaux 20 sont maintenus en position contractée par la gaine interne 36.

La gaine interne 36 est ensuite rétractée axialement à l'écart de la tête 34 par rapport à la gaine intermédiaire 24, et par rapport au tuteur 32, pour découvrir les bras distaux 20, qui se déploient alors. L'outil 19 est alors déplacé vers le ventricule gauche pour plaquer les bras distaux 20 contre la face atriale des feuillets 13.

Une fois le manchon distal 16 en place, la gaine interne 36 est rétractée de manière à libérer ce manchon distal 16. Ce manchon distal 16 étant disposé à l'intérieur du manchon proximal 14, il est maintenu en configuration contractée par ce manchon proximal 14, lui-même maintenu en configuration contractée par la gaine intermédiaire 24.

En se déployant à l'intérieur du manchon proximal 14, le manchon distal 16 se lie à ce manchon proximal 14.

On notera que, dans cette position, les feuillets 13 de la valve native sont intercalés entre le manchon distal 16 et les bras proximaux 18.

Une fois les manchons proximal 14 et distal 16 ainsi liés, on rétracte la gaine intermédiaire 24, de manière à libérer le manchon proximal 14. Le manchon proximal 14 se déploie radialement alors jusqu'à sa configuration déployée, ainsi que le manchon distal 16 à l'intérieur de ce manchon proximal 14 déployé.

Dans cette configuration déployée du manchon proximal 14, les moyens de maintien 22 relâchent les bras proximaux 18, qui sont alors rappelés élastiquement vers leur position d'ancrage, comme décrit précédemment.

Ceci étant fait, la fixation axiale entre le tuteur 32 et l'extrémité proximale de l'implant 10 est libérée. Le tuteur 32, la tête 34 et la tige sont alors retirés hors du patient à travers le conduit interne.

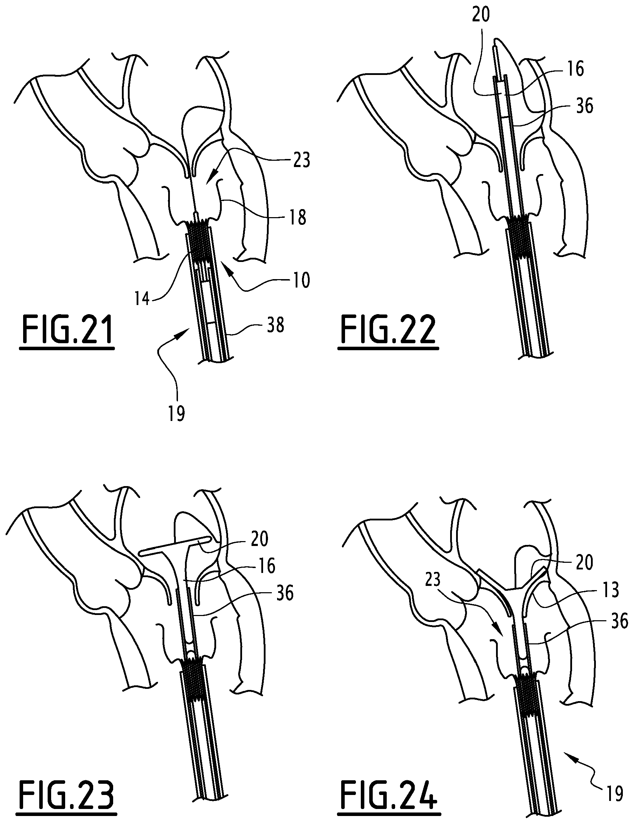

On peut également prévoir d'autres procédés de largage. Par exemple, un autre procédé de largage est décrit en référence aux figures 21 à 26.

Le procédé de largage comporte l'introduction d'une extrémité distale de l'outil de largage 19, comprenant l'implant 10 dans la configuration contractée, dans la cavité ventriculaire. Ensuite, comme cela est représenté sur la figure 21, la gaine extérieure 38 est rétractée axialement de manière à libérer les bras proximaux 18, comme décrit précédemment. Le déploiement des bras proximaux 18 définit un espace de réception 23, qui est alors entièrement libre.

Il convient de noter que les bras proximaux 18 sont déployés avant que l'armature 12 soit formée, c'est-à-dire avant que le manchon proximal 14 soit assemblé avec le manchon distal 16.

Ensuite, comme cela est représenté sur la figure 22, la gaine intérieure 36, contenant le manchon distal 16, est déplacé axialement jusqu'à la cavité atriale, à travers l'anneau mitral, de sorte que les bras distaux 20 soient positionnés dans la cavité atriale.

Puis, comme cela est représenté sur la figure 23, la gaine intérieure 36 est rétractée axialement de manière à libérer les bras distaux 20, qui sont alors déployés. Durant cette étape, le manchon atrial 16 reste partiellement contracté dans la gaine intérieure 36.

Comme cela est représenté sur la figure 24, l'outil 19 est ensuite déplacé vers le ventricule gauche pour appliquer les bras distaux 20 contre la face atriale des feuillets 13. Ainsi, les bras distaux 20 appliquent un effort axial contre la face atriale des feuillets 13, cet effort axial étant orienté depuis la cavité atriale vers la cavité ventriculaire.

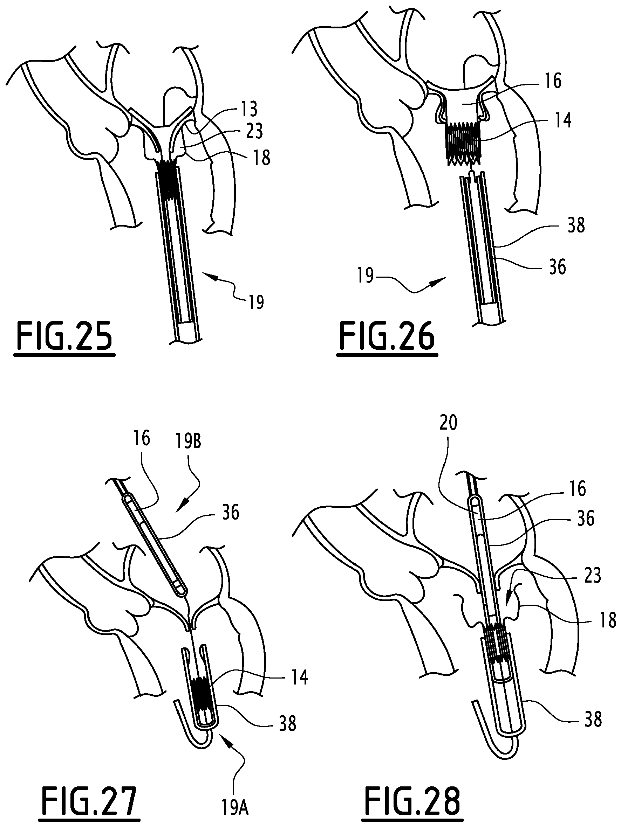

Puis, l'espace de réception 23 est positionné en regard des feuillets 13, de sorte que ces feuillets 13 sont insérés dans cet espace de réception 23 lorsque l'outil 19 est déplacé vers ces feuillets 13, comme représenté sur la figure 25.

Ainsi, les bras proximaux 18 sont appliqués contre la face ventriculaire des feuillets 13. Ainsi, les bras proximaux 18 appliquent un effort axial contre la face ventriculaire des feuillets 13, cet effort axial étant orienté depuis la cavité ventriculaire vers la cavité atriale. La direction de l'effort axial appliqué par les bras proximaux 18 est donc opposée à la direction de l'effort axial appliqué par les bras distaux 20.

Comme indiqué précédemment, le manchon proximal 14 est mobile axialement relativement au manchon distal 16, si bien que la position relative du manchon proximal 14 par rapport au manchon distal 16 peut être choisie en fonction de la configuration du passage de circulation de sang dans lequel l'implant est installé.

La position du manchon proximal 14 est ajustée, puis la gaine intérieure 36 et la gaine extérieure 38 sont rétractées, de sorte que le manchon distal 16 et le manchon proximal 14 sont déployés, comme représenté sur la figure 26. Il est à noter que le manchon distal 16 et le manchon proximal 14 peuvent être déployés simultanément, ou en variante le manchon distal 16 est déployé à l'intérieur du manchon proximal 14 préalablement au déploiement de ce manchon proximal 14.

Après le déploiement des manchons proximal 14 et distal 16, ces manchons proximal 14 et distal 16 sont assemblés, formant ainsi l'armature 12. En d'autres termes, l'armature 12 n'est formée que dans la configuration déployée.

Une fois ces déploiements effectués, l'outil de largage est retiré hors du patient.

Il est également possible de prévoir d'autres procédés d'installation de l'implant.

En particulier, le manchon proximal 14 et le manchon distal 16 formant deux ensembles distincts, il est possible de les amener dans le passage de circulation de sang via deux voies d'accès distinctes.

Par exemple, le manchon distal 16 peut être amené par voie antérograde transveineuse, et ainsi introduit dans la cavité atriale sans passer par la cavité ventriculaire, et le manchon proximal 14 peut être amené par voie rétrograde transaortique, et ainsi introduit dans la cavité ventriculaire sans passer par la cavité atriale.

Dans ce cas, le dispositif de traitement comporte un premier outil de largage pour le manchon proximal 14 et un second outil de largage pour le manchon distal 16.

Ces premier et second outils de largage présentent des diamètres inférieurs au diamètre d'un outil de largage portant à la fois le manchon proximal 14 et distal 16. En d'autres termes, dans ce procédé d'installation, on prévoit deux voies d'accès plus étroites que l'unique voie d'accès nécessaire pour les procédés d'installation précédemment décrits.

Un tel procédé d'installation via deux voies d'accès distinctes est décrit en référence aux figures 27 à 32.

Tout d'abord, une première partie 19A de l'outil de largage, comprenant une gaine extérieure 38 et le manchon proximal 14 en configuration contractée à l'intérieur de cette gaine extérieure 38, sont introduits dans la cavité ventriculaire par une première voie.

Ensuite, comme cela est représenté sur la figure 27, une seconde partie 19B de l'outil de largage, comprenant une gaine intérieure 36 et le manchon distal 16 en configuration contractée à l'intérieur de cette gaine intérieure 36, sont introduits dans la cavité atriale par une seconde voie différente de ladite première voie.

Ainsi, conformément à ce procédé d'installation, la seconde partie 19B de l'outil de largage n'est pas insérée à travers l'anneau mitral dans la cavité atriale.

La gaine extérieure 38 est ensuite rétractée axialement de manière à relâcher les bras proximaux 18, qui sont ainsi déployés comme décrit précédemment. Le déploiement des bras proximaux 18 définit ainsi l'espace de réception 23.

Ensuite, comme cela est représenté sur la figure 28, la gaine intérieure 36 contenant le manchon distal 16 est déplacée axialement jusqu'à la cavité ventriculaire, au-delà de l'anneau mitral, de sorte que les bras distaux 20 restent positionnés dans la cavité atriale, et que le manchon distal 16 soit positionné en partie dans la cavité ventriculaire.

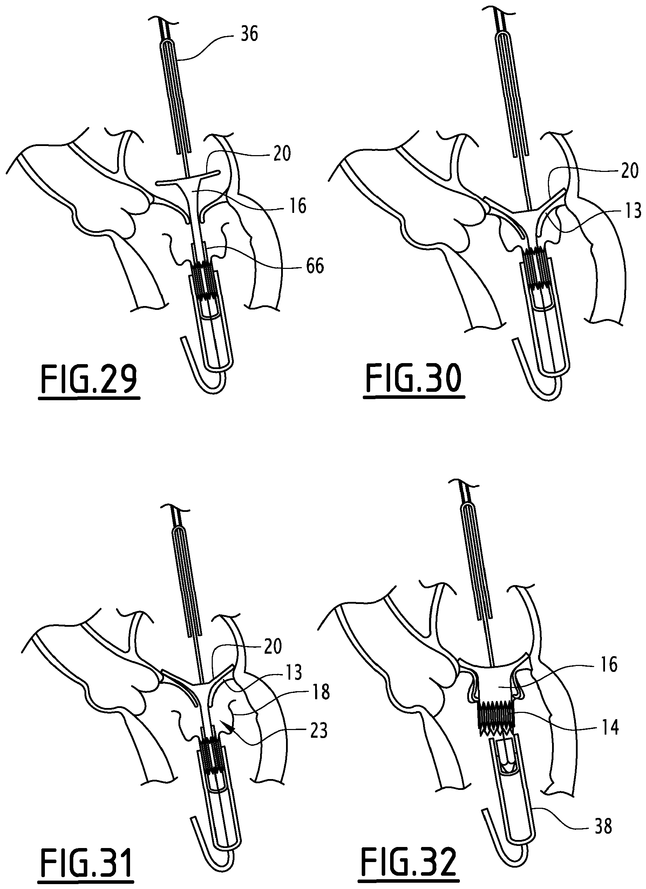

Ensuite, comme cela est représenté sur la figure 29, la gaine intérieure 36 est rétractée axialement afin de libérer les bras distaux 20, qui se déploient alors. Durant cette étape, le manchon distal 16 reste partiellement contracté, par exemple grâce à un élément annulaire de maintien 66 entourant ce manchon distal 16.

Les bras distaux 20 sont ensuite appliqués contre la face atriale des feuillets 13, comme cela est représenté sur la figure 30. Ainsi, les bras distaux 20 appliquent un effort axial contre la face atriale des feuillets 13, cet effort axial étant orienté depuis la cavité atriale vers la cavité ventriculaire.

L'espace de réception 23 est ensuite positionné en regard des feuillets 13, de sorte que ceux-ci sont insérés dans cet espace de réception 23 lorsque la première partie 14A de l'outil de largage est déplacée vers ces feuillets 13, comme représenté sur la figure 31. Puis, l'espace de réception 23 est positionné en regard des feuillets 13, de sorte que ces feuillets 13 sont insérés dans cet espace de réception 23 lorsque l'outil 19 est déplacé vers ces feuillets 13, comme représenté sur la figure 25.

Ainsi, les bras proximaux 18 sont appliqués contre la face ventriculaire des feuillets 13. Ainsi, les bras proximaux 18 appliquent un effort axial contre la face ventriculaire des feuillets 13, cet effort axial étant orienté depuis la cavité ventriculaire vers la cavité atriale. La direction de l'effort axial appliqué par les bras proximaux 18 est donc opposée à la direction de l'effort axial appliqué par les bras distaux 20.

Comme indiqué précédemment, le manchon proximal 14 est mobile axialement relativement au manchon distal 16, si bien que la position relative du manchon proximal 14 par rapport au manchon distal 16 peut être choisie en fonction de la configuration du passage de circulation de sang dans lequel l'implant est installé.

La position du manchon proximal 14 est ajustée, puis l'élément annulaire de maintien 66 et la gaine extérieure 38 sont rétractés, de sorte que le manchon distal 16 et le manchon proximal 14 sont déployés, comme représenté sur la figure 32.

Il est à noter que le manchon distal 16 et le manchon proximal 14 peuvent être déployés simultanément, ou en variante le manchon distal 16 est déployé à l'intérieur du manchon proximal 14 préalablement au déploiement de ce manchon proximal 14.

Après le déploiement des manchons proximal 14 et distal 16, ces manchons proximal 14 et distal 16 sont assemblés, formant ainsi l'armature 12. En d'autres termes, l'armature 12 n'est formée que dans la configuration déployée.

Une fois ces déploiements effectués, l'outil de largage est retiré hors du patient, chacune de ses parties à travers la voie correspondante.

## Revendications

1. Implant (10) destiné à être placé dans un passage de circulation de sang, et à être fixé sur un tissu (13), et comprenant :
- un manchon proximal (14), de forme générale tubulaire autour d'un axe central (X), s'étendant longitudinalement entre une extrémité proximale (14A) et une extrémité distale (14B), le manchon proximal (14) étant déployable entre une configuration contractée et une configuration déployée,
- une pluralité de bras proximaux (18), s'étendant chacun entre une première extrémité (18A) liée à l'extrémité distale (14B) du manchon proximal (14), et une seconde extrémité (18B) libre destinée à s'appuyer sur une première face du tissu (13), chaque bras proximal (18) s'étendant dans la direction de l'axe central (X) de sorte que son extrémité libre (18B) soit disposée au-delà de l'extrémité distale (14B) du manchon proximal (14),
- un manchon distal (16), de forme générale tubulaire autour de l'axe central (X), déployable entre une configuration contractée et une configuration déployée, destiné à être assemblé avec le manchon proximal (14) pour former ensemble une armature tubulaire (12), cette armature (12) définissant un conduit interne de circulation du sang lorsque le manchon proximal (14) et le manchon distal (16) sont assemblés et chacun en configuration déployée,
- une pluralité de bras distaux (20), portés par le manchon distal (16), et s'étendant sensiblement perpendiculairement à l'axe central (X) dans la configuration déployée, destinés à s'appuyer sur une deuxième face du tissu (13), de sorte que le tissu (13) soit alors pincé entre les bras proximaux (18) et les bras distaux (20),
**caractérisé en ce que** :
- au moins un bras proximal (18) est déformable élastiquement, entre une position d'écartement et une position d'ancrage, telles que, en l'absence de sollicitation extérieure, la distance radiale entre son extrémité libre (18B) et son extrémité liée (18A) est supérieure dans la position d'écartement plutôt que dans la position d'ancrage, ce bras proximal (18) étant rappelé élastiquement vers sa position d'ancrage,
- l'implant (10) comporte des moyens (22) de maintien dudit au moins un bras proximal déformable (18) dans sa position d'écartement, comprenant :
• une première butée (22A), portée par ce bras proximal (18), et
• une seconde butée (22B), destinée à coopérer avec la première butée (22A) lorsque le manchon proximal (14) est en configuration contractée pour maintenir ce bras proximal (18) dans sa position d'écartement, et à libérer cette première butée (22A) lorsque le manchon proximal (14) est en configuration déployée pour laisser le bras proximal (18) se déplacer vers sa position d'ancrage.

2. Implant (10) selon la revendication 1, dans lequel la première butée (22A) du bras proximal (18) s'étend latéralement par rapport à ce bras proximal (18), cette première butée (22A) étant de préférence agencée à proximité de l'extrémité liée (18A) de ce bras proximal (18).

3. Implant (10) selon la revendication 1 ou 2, dans lequel la seconde butée (22B) est portée par le manchon proximal (14).

4. Implant (10) selon la revendication 3, dans lequel le manchon proximal (14) est formé d'éléments filiformes (15) disposés en grillage, formant des mailles (M), par exemple en losange, chaque bras proximal (18) muni d'une première butée (22A) étant agencé circonférentiellement sur le manchon proximal (14) entre deux mailles (M) consécutives, la seconde butée (22B) correspondante étant portée par au moins l'un des éléments filiformes (15) formant ces deux mailles (M) consécutives, ces éléments filiformes (15) étant rapprochés l'un de l'autre en configuration contractée, de sorte que la seconde butée (22B) coopère avec la première butée (22A), et éloignés l'un de l'autre en configuration déployée, de façon à laisser un passage radial pour la première butée (22A), libérant ainsi cette première butée (22A).

5. Implant (10) selon l'une quelconque des revendications précédentes, dans lequel le manchon proximal (14) porte au moins deux bras proximaux (18) munis chacun d'une première butée (22A), tels que la distance entre la première butée (22A) et l'extrémité liée (18A) de l'un de ces deux bras proximaux (18) est supérieure à la distance entre la première butée (22A) et l'extrémité liée (18A) de l'autre de ces deux bras proximaux (18).

6. Implant (10) selon l'une quelconque des revendications 1 à 5, dans lequel la première butée (22A) d'au moins l'un des bras proximaux (18) est formée par une partie en saillie de ce bras proximal (18), s'étendant latéralement par rapport à ce bras proximal (18).

7. Implant (10) selon l'une quelconque des revendications 1 à 5, dans lequel la première butée (22A) d'au moins l'un des bras proximaux (18) est formée par un élément (25) rapporté sur ce bras proximal (18), par exemple soudé, cet élément (25) s'étendant latéralement par rapport à ce bras proximal (18).

8. Dispositif de traitement d'un passage de circulation de sang, **caractérisé en ce qu'**il comporte :
- un implant (10) selon l'une quelconque des revendications précédentes ; et
- un outil (19) de largage de l'implant (10), les manchons proximal (14) et distal (16) étant montés dans leurs configurations contractées dans l'outil de largage (19).

9. Dispositif de traitement selon la revendication 8, dans lequel :
- l'outil de largage (19) comporte une gaine (26) de maintien de chaque bras proximal (18) muni d'une première butée (22A) dans sa position d'écartement,
- ladite gaine de maintien (26) présente une forme générale de révolution autour de l'axe longitudinal (X), et est destinée à être disposée autour du manchon proximal (18) en configuration contractée,
- ladite gaine de maintien (26) comporte des bandes longitudinales (30) séparées par des ouvertures longitudinales (28), chaque bande longitudinale (30) étant destinée à passer à travers une ouverture prévue dans un bras proximal (18) respectif,
- chaque bande longitudinale (30) comporte une seconde butée (22B) destinée à coopérer avec la première butée (22A) du bras proximal (18) à travers lequel passe cette bande longitudinale (30).

10. Dispositif de traitement selon la revendication 9, dans lequel ladite bande longitudinale (30) est munie d'un bossage, faisant saillie radialement vers l'extérieur de la gaine de maintien (26), portant ladite seconde butée (22B).

## Patentansprüche

1. Implantat (10), das dazu bestimmt ist, in einer Blutkreislaufbahn angeordnet zu werden und an einem Gewebe (13) befestigt zu werden, und Folgendes umfasst:
- eine proximale Muffe (14) im Allgemeinen in Röhrenform um eine Mittelachse (X), die sich longitudinal zwischen einem proximalen Ende (14A) und einem distalen Ende (14B) erstreckt, wobei die proximale Muffe (14) zwischen einer zusammengezogenen Konfiguration und einer entfalteten Konfiguration entfaltbar ist,
- mehrere proximale Arme (18), die sich jeweils zwischen einem ersten Ende (18A), das mit dem distalen Ende (14B) der proximalen Muffe (14) verbunden ist, und einem freien zweiten Ende (18B), das dazu bestimmt ist, sich an einer ersten Fläche des Gewebes (13) abzustützen, erstrecken, wobei sich jeder proximale Arm (18) in Richtung der Mittelachse (X) erstreckt, derart, dass sein freies Ende (18B) jenseits des distalen Endes (14B) der proximalen Muffe (14) angeordnet ist,
- eine distale Muffe (16) im Allgemeinen in Röhrenform um die Mittelachse (X), die zwischen einer zusammengezogenen Konfiguration und einer entfalteten Konfiguration entfaltbar ist und dazu bestimmt ist, mit der proximalen Muffe (14) zusammengefügt zu werden, um zusammen eine röhrenförmige Ummantelung (12) zu bilden, wobei diese Ummantelung (12) eine innere Blutkreislaufleitung definiert, wenn die proximale Muffe (14) und die distale Muffe (16) zusammengefügt sind und jede von ihnen in der entfalteten Konfiguration ist,
- mehrere distale Arme (20), die von der distalen Muffe (16) getragen werden und die sich in der entfalteten Konfiguration im Wesentlichen senkrecht zu der Mittelachse (X) erstrecken und dazu bestimmt sind, sich an einer zweiten Fläche des Gewebes (13) abzustützen, derart, dass das Gewebe (13) dann zwischen den proximalen Armen (18) und den distalen Armen (20) eingeklemmt ist,
**dadurch gekennzeichnet, dass**:
- wenigstens ein proximaler Arm (18) zwischen einer beabstandeten Position und einer Verankerungsposition elastisch verformbar ist, derart, dass bei Abwesenheit einer äußeren Kraft der radiale Abstand zwischen seinem freien Ende (18B) und seinem verbundenen Ende (18A) in der beabstandeten Position größer als in der Verankerungsposition ist, wobei dieser proximale Arm (18) in seine Verankerungsposition elastisch zurückgestellt wird,
- das Implantat (10) Mittel (22) zum Halten des wenigstens einen verformbaren proximalen Arms (18) in seiner beabstandeten Position umfasst, die ihrerseits umfassen:
• einen ersten Anschlag (22A), der von diesem proximalen Arm (18) getragen wird, und
• einen zweiten Anschlag (22B), der dazu bestimmt ist, mit dem ersten Anschlag (22A) zusammenzuwirken, wenn die proximale Muffe (14) in der zusammengezogenen Konfiguration ist, um diesen proximalen Arm (18) in seiner beabstandeten Position zu halten, und diesen ersten Anschlag (22A) freizugeben, wenn die proximale Muffe (14) in der entfalteten Konfiguration ist, damit sich der proximale Arm (18) in seine Verankerungsposition verlagern kann.

2. Implantat (10) nach Anspruch 1, wobei sich der erste Anschlag (22A) des proximalen Arms (18) seitlich in Bezug auf diesen proximalen Arm (18) erstreckt, wobei dieser erste Anschlag (22A) vorzugsweise in der Nähe des verbundenen Endes (18A) dieses proximalen Arms (18) angeordnet ist.

3. Implantat (10) nach Anspruch 1 oder 2, wobei der zweite Anschlag (22B) von der proximalen Muffe (14) getragen wird.

4. Implantat (10) nach Anspruch 3, wobei die proximale Muffe (14) aus gitterförmig angeordneten drahtförmigen Elementen (15) gebildet ist, die Maschen (M) beispielsweise in Rautenform bilden, wobei jeder proximale Arm (18), der mit einem ersten Anschlag (22A) versehen ist, in Umfangsrichtung auf der proximalen Muffe (14) zwischen zwei aufeinanderfolgenden Maschen (M) angeordnet ist, wobei der entsprechende zweite Anschlag (22B) von wenigstens einem der drahtförmigen Elemente (15), die diese zwei aufeinanderfolgenden Maschen (M) bilden, getragen wird, wobei diese drahtförmigen Elemente (15) in der zusammengezogenen Konfiguration einander angenähert sind, derart, dass der zweite Anschlag (22B) mit dem ersten Anschlag (22A) zusammenwirkt, und in der entfalteten Konfiguration voneinander entfernt sind, derart, dass ein radialer Durchlass für den ersten Anschlag (22A) frei bleibt, wodurch dieser erste Anschlag (22A) freigegeben wird.

5. Implantat (10) nach einem der vorhergehenden Ansprüche, wobei die proximale Muffe (14) wenigstens zwei proximale Arme (18) trägt, wovon jeder mit einem ersten Anschlag (22A) versehen ist, derart, dass der Abstand zwischen dem ersten Anschlag (22A) und dem verbundenen Ende (18A) eines dieser zwei proximalen Arme (18) größer als der Abstand zwischen dem ersten Anschlag (22A) und dem verbundenen Ende (18A) des anderen dieser zwei proximalen Arme (18) ist.

6. Implantat (10) nach einem der Ansprüche 1 bis 5, wobei der erste Anschlag (22A) wenigstens eines der proximalen Arme (18) durch einen vorstehenden Teil dieses proximalen Arms (18) gebildet ist und sich in Bezug auf diesen proximalen Arm (18) seitlich erstreckt.

7. Implantat (10) nach einem der Ansprüche 1 bis 5, wobei der erste Anschlag (22A) wenigstens eines der proximalen Arme (18) durch Element (25) gebildet ist, das an diesem proximalen Arm (18) beispielsweise durch Schweißen angefügt ist, wobei sich dieses Element (25) seitlich in Bezug auf diesen proximalen Arm (18) erstreckt.

8. Vorrichtung zum Bearbeiten eines Blutkreislaufdurchlasses, **gekennzeichnet durch**:
- ein Implantat (10) nach einem der vorhergehenden Ansprüche; und
- ein Werkzeug (19) zum Ausgeben des Implantats (10), wobei die proximale Muffe (14) und die distale Muffe (16) in dem Ausgabewerkzeug (19) in ihren zusammengezogenen Konfigurationen montiert sind.

9. Bearbeitungsvorrichtung nach Anspruch 8, wobei:
- das Ausgabewerkzeug (19) eine Hülse (26) umfasst, um jeden proximalen Arm (18), der mit einem ersten Anschlag (22A) versehen ist, in seiner beabstandeten Position zu halten, und
- die Haltehülse (26) eine im allgemeinen rotationssymmetrische Form um die Längsachse (X) aufweist und dazu bestimmt ist, um die proximale Muffe (18) in der zusammengezogenen Konfiguration angeordnet zu werden,
- die Haltehülse (26) longitudinale Bänder (30) aufweist, die durch longitudinale Öffnungen (28) getrennt sind, wobei jedes longitudinale Band (30) dazu bestimmt ist, sich durch eine in einem jeweiligen proximalen Arm (18) vorgesehene Öffnung zu bewegen,
- jedes longitudinale Band (30) einen zweiten Anschlag (22B) aufweist, der dazu bestimmt ist, mit dem ersten Anschlag (22A) des proximalen Arms (18), durch den sich dieses longitudinale Band (30) bewegt, zusammenzuwirken.

10. Bearbeitungsvorrichtung nach Anspruch 9, wobei das longitudinale Band (30) mit einer Erhebung versehen ist, die radial in die äußere Umgebung der Haltehülse (26) vorsteht und den zweiten Anschlag (22B) trägt.

## Claims

1. An implant (10) designed to be placed in a blood circulation passage, and to be fastened on a tissue (13), and comprising:
- a proximal sleeve (14), generally tubular around a central axis (X), extending longitudinally between a proximal end (14A) and a distal end (14B), the proximal sleeve (14) being deployable between a contracted configuration and a deployed configuration,
- a plurality of proximal arms (18), each extending between a first end (18A) connected to the distal end (14B) of the proximal sleeve (14), and a second free end (18B) designed to bear on a first face of the tissue (13), each proximal arm (18) extending in the direction of the central axis (X) such that its free end (18B) is positioned beyond the distal end (18B) of the proximal sleeve (14),
- a distal sleeve (16), with a generally tubular shape around the central axis (X), deployable between a contracted configuration and a deployed configuration, designed to be assembled with the proximal sleeve (14) to form a tubular armature (12) together, that armature (12) defining an internal blood circulation conduit when the proximal sleeve (14) and the distal sleeve (16) are assembled and each in the deployed configuration,
- a plurality of distal arms (20), supported by the distal sleeve (16), and extending substantially perpendicular to the central axis (X) in the deployed configuration, designed to bear on a second face of the tissue (13), such that the tissue (13) is then pinched between the proximal arms (18) and the distal arms (20),
**characterized in that**:
- at least one proximal arm (18) is elastically deformable, between a separating position and an anchoring position, such that, when there is no outside bias, the radial distance between its free end (18B) and its connected end (18A) is larger in the separated position rather than in the anchoring position, this proximal arm (18) being elastically returned toward its anchoring position,
- the implant (10) includes means (22) for maintaining said at least one deformable proximal arm (18) in its separating position, comprising:
• a first stop (22A), supported by that proximal arm (18), and
• a second stop (22B), designed to cooperate with the first stop (22A) when the proximal sleeve (14) is in the contracted configuration to keep that proximal arm (18) in its separating position, and to release that first stop (22A) when the proximal sleeve (14) is in the deployed configuration to allow the proximal arm (18) to move toward its anchoring position.

2. The implant (10) according to claim 1, wherein the first stop (22A) of the proximal arm (18) extends laterally relative to that proximal arm (18), the first stop (22A) preferably being arranged near the connected end (18A) of that proximal arm (18).

3. The implant (10) according to claim 1 or 2, wherein the second stop (22B) is supported by the proximal sleeve (14).

4. The implant (10) according to claim 3, wherein the proximal sleeve (14) is formed by different filiform elements (15) arranged in a grid, forming cells (M), for example diamond-shaped cells, each proximal arm (18) provided with a first stop (22A) being arranged circumferentially on the proximal sleeve (14) between two consecutive cells (M), the corresponding second stop (22B) being supported by at least one of the filiform elements (15) forming those two consecutive cells (M), these filiform elements (15) being brought closer to one another in the contracted configuration, such that the second stop (22B) cooperates with the first stop (22A), and separated from one another in the deployed configuration, so as to leave a radial passage for the first stop (22A), thus releasing that first stop (22A).

5. The implant (10) according to any one of the preceding claims, wherein the proximal sleeve (14) supports at least two proximal arms (18) each provided with a first stop (22A), such that the distance between the first stop (22A) and the connected end (18A) of one of those two proximal arms (18) is greater than the distance between the first stop (22A) and the connected end (18A) of the other of those two proximal arms (18).

6. The implant (10) according to any one of claims 1 to 5, wherein the first stop (22A) of at least one of the proximal arms (18) is formed by a protruding part of that proximal arm (18), extending laterally relative to that proximal arm (18).

7. The implant (10) according to any one of claims 1 to 5, wherein the first stop (22A) of at least one of the proximal arms (18) is formed by an element (25) attached on that proximal arm (18), for example welded, that element (25) extending laterally relative to that proximal arm (18).

8. A treatment device for a blood circulation passage, **characterized in that** it includes:
- an implant (10) according to any one of the preceding claims; and
- a tool (19) for releasing the implant (10), the proximal (14) and distal (16) sleeves being mounted in their contracted configurations in the release tool (19).

9. The treatment device according to claim 8, wherein:
- the release tool (19) includes a sheath (26) for maintaining each proximal arm (18) provided with a first stop (22A) in its separating position,
- said maintaining sheath (26) has a general shape of revolution around the longitudinal axis (X), and is designed to be positioned around the proximal sleeve (18) in the contracted configuration,
- said maintaining sheath (26) includes longitudinal strips (30) separated by longitudinal openings (28), each longitudinal strip (30) being designed to pass through an opening provided in a respective proximal arm (18),
- each longitudinal strip (30) includes a second stop (22B) designed to cooperate with the first stop (22A) of the proximal arm (18) through which that longitudinal strip (30) passes.

10. The treatment device according to claim 9, wherein said longitudinal strip (30) is provided with a boss, protruding radially toward the outside of the maintaining sheath (26), bearing said second stop (22B).
